# EUROPEAN PATENT SPECIFICATION

(11) **EP 1 699 502 B1**
(45) Date of publication and mention of the grant of the patent: **21.05.2014**
(21) Application number: 04813158.5
(22) Date of filing: 03.12.2004
(51) Int. Cl.: A61L 29/14, A61M 25/10, A61M 25/00

(54) **MEDICAL BALLOON WITH VARYING PHYSICAL PROPERTIES AND METHOD FOR FORMING SAME**
MEDIZINISCHER BALLON MIT UNTERSCHIEDLICHEN PHYSIKALISCHEN EIGENSCHAFTEN UND FORMUNGSVERFAHREN DAFÜR
BALLONNET MEDICAL A PROPRIETES PHYSIQUES VARIABLES ET SON PROCEDE DE FABRICATION

(30) Priority: 31.12.2003 US 749821; 05.02.2004 US 772477
(43) Date of publication of application: 13.09.2006
(73) Proprietor: Boston Scientific Limited, St. Michael, Barbados, West Indies (BB)
(72) Inventor: BURGMEIER, Robert, Plymouth, Minnesota 55447 (US); GOODIN, Richard, Blaine, Minnesota 55449 (US); DELANEY, Joseph, Jr., Minneapolis, Minnesota 55407 (US); PETERSON, Larry, Champlin, Minnesota 55316 (US)
(74) Representative: Lang, Johannes
(86) International application number: PCT/US2004/040797
(87) International publication number: WO 2005/065735

(56) References cited:
- EP-A- 0 376 656
- WO-A-03/051421
- US-A- 5 316 706
- DATABASE WPI Section Ch, Week 200365 Derwent Publications Ltd., London, GB; Class A32, AN 2003-682929 XP002326272 & JP 2003 062081 A (ASAHI INTECH KK) 4 March 2003 (2003-03-04)

## Description

### BACKGROUND OF THE INVENTION

Various medical devices, such as catheters, tubes, balloons, stents and the like, are known to have physical performance requirements which change at particular points, or ranges of area or length. For instance, catheters typically need to be soft and flexible toward the distal end while at the same time becoming much more rigid and kink resistant proximally in order to effectively transmit torque and crossing forces from their proximal ends to the distal tip.

Medical devices comprising catheter balloons are used in an increasingly widening variety of applications including vascular dilatation, stent delivery, drug delivery, delivery and operation of sensors and surgical devices such as blades, and the like. The desired physical property profile for the balloons used in these devices vary according to the specific application, but for many applications a high strength robust balloon is necessary and good softness and trackability properties are highly desirable.

Commercial catheter balloons have been formed of a wide variety of polymeric materials, including PET, nylons, polyurethanes, polyolefins, and various block copolymer thermoplastic elastomers.

US 4490421, Levy, and US 5264260, Saab, describe PET balloons. US 4906244, Pinchuk et al, and US 5328468, Kaneko, describe polyamide balloons. US 4950239, Gahara, and US 5500180, Anderson et al describe balloons made from polyurethane block copolymers. US 5556383, Wang et al and US 6146356, Wang et al, describes balloons made from polyether-block-amide copolymers and polyester-block-ether copolymers. US 6270522, Simhambhatla, et al, describes balloons made from polyester-block-ether copolymers of high flexural modulus. US 5344400, Kaneko, describes balloons made from polyarylene sulfide. All of these balloons are produced from extruded tubing of the polymeric material by a blow-forming radial expansion process. US 5250069, Nobuyoshi et al, US 5797877, Hamilton et al, and US 5270086, Hamlin, mention still further materials which may be used to make such balloons.

It has been found that polymers with a high content of butylene terephthalate can crystallize so extensively from an extrusion melt that balloon formation from an extruded parison is very difficult, if possible. A solution to this problem, taught in US 6465067, Wang et al, is to add boric acid to the polymer composition.

In commonly owned copending US application 10/055747, medical devices formed of thermoplastic polymers containing chain extension additives which increase polymer molecular weight are described.

In commonly owned copending US application 10/087653, filed Feb. 28, 2002, incorporated herein by reference, it is disclosed that improved balloon properties can be obtained by controlling the parison extrusion in a manner which restricts the elongation of the parison material in the longitudinal direction. The application discloses that decreasing the gap between the extrusion head and the cooling bath tank can lower parison elongation by shortening the quench time.

In commonly owned copending US application 10/617428, filed July 10, 2003, it is taught that varying the cooling tank gap during an extrusion can provide a catheter tube or balloon parison which has variable properties along its length.

In a balloon catheter, heat welded balloon-to-tube bonds, typically provided by laser heating, are commonly used for their high reliability. However, heat welded bonds provide a new problem, the melted or softened regions of the joined parts will often resolidify relatively slowly, allowing crystallization to develop with consequent increased stiffness. At the distal end of the catheter where the balloon is typically bonded to the catheter inner tube, the increased crystallinity in the bond can adversely affect the desired softness and trackability and of the catheter tip. Selecting a slow crystallizing polymer for the balloon material is usually not a suitable option since balloon material selection and processing steps are typically directed to maximizing balloon wall strength and hence providing a high degree of crystallization.

At the same time the catheter distal outer tube near the site, where it is bonded to the proximal waist of the balloon, often is subjected to very high tensile stress when the balloon is collapsed after use and is being withdrawn into a guide catheter or a protective sleeve. In some cases, particularly with larger balloons, the catheter shaft immediately proximal of the balloon may begin to yield before the balloon is successfully withdrawn. Consequently the tensile strength of the catheter outer can limit the minimum guide catheter or sleeve diameter which may be used with the catheter.

WO 2003/051 421, EP 0 376 656 A1, and US 5 316 706 A disclose different medical devices formed of thermoplastic polymers. JP 2003 062 081 A discloses medical balloons with modified crystallinity.

### SUMMARY OF THE INVENTION

The present invention is directed to medical balloons that are formed of thermoplastic material or materials, and to methods of forming such balloons. In particular it is directed to such balloons in which a melt processed part desirably has different crystallizing properties at different locations. In accordance with the invention the part is formed of a polymer composition by inclusion a of polymer crystallization modifier in the composition making up at least a portion of such part, the amount of the polymer crystallization modifier being varied in the part in accordance with the desired difference in crystallization behavior.

In one embodiment a tubular balloon parison is extruded with a composition which varies in composition by localized inclusion of a crystallization inhibitor. The portion of the parison which forms the distal waist of the balloon may be provided with such inhibitor in order to reduce the rigidity which develops upon heat welding of the balloon to the distal inner tube of the catheter.

Further aspects of the invention are described in the following detailed description of the invention or in the claims.

### BRIEF DESCRIPTION OF THE DRAWING

Figure 1 is a schematic diagram of an extruded tubular balloon parison prepared in accordance with one embodiment of the present invention.
Figure 2 is a cross-sectional view of a tubular member in which the polymer composition is varied step-wise through the thickness dimension, in accordance with another embodiment of the invention.
Figure 3 is a perspective view of a tubular balloon parison prepared in accordance with a still further embodiment of the invention.

### DETAILED DESCRIPTION OF THE INVENTION

Medical device parts to which the invention may be applied include tubes, cannulae, catheter shafts, balloons and parisons therefor, stents, connectors, leads, or parts of any such devices. A part may be the entire device or a discretely formed portion thereof.

The medical balloon part is formed of melt processed polymer material. The polymer material is formed of a polymer material composition which comprises a thermoplastic polymer or mixture thereof. In at least a portion of the part, the polymer composition further comprises a crystallization modifier. From a first portion of the inventive part to a second portion thereof, the composition is varied in the amount of crystallization modifier employed therein.

The invention may be used with any known semi-crystalline thermoplastic materials. Such materials may include olefin polymers and copolymers, acrylic, styrenic and vinyl polymers (e.g. poly(vinyl chloride)) and copolymers; polyethers; polyurethanes; polyesters and copolyesters; polycarbonates; thermoplastic elastomers; silicone-polycarbonate copolymers; polyamides; thermoplastic polyimides; liquid crystal polymers; ABS (acrylonitrile butadiene styrene); ANS (acrylonitrile styrene); Delrin polyacetal; PEI (polyetherimide); polyetheretherketone (PEEK) and polyether sulfone (PES). Film forming polymers may be used.

Olefin polymers and copolymers include irradiated polyethylene, polypropylene, ultra-high molecular weight polyolefins, low, linear low, medium and high density polyethylenes; polypropylenes; poly(ethylene vinyl acetate) (EVA); poly(ethylene vinyl alcohol) (EVOH) and EVA/EVOH terpolymers; ethylene-butylene-styrene block copolymers blended with low molecular weight polystyrene and, optionally, polypropylene, and similar compositions substituting butadiene or isoprene in place of the ethylene and butylene, and olefin ionomers (copolymers of olefin monomers and a metal salt of an olefinic acid, such as (meth)acrylic acid, succinic acid, maleic acid or fumaric acid).

Orientable polyesters, especially polyethylene terephthalate (PET), are among materials for forming catheter balloons. Suitable PET polymers have an initial intrinsic viscosity of at least 0.5, for instance, 0.6-1.3. Other high strength polyester materials, such as poly(ethylene napthalenedicarboxylate) (PEN), polytrimethylene terephthalate (PTT) and poly(butylene terephthalate) (PBT) may also be used. Polyester copolymers may also be employed, for instance, the random copolymers made from dimethyl terephthalate, dimethyl isophthalate and ethylene glycol described in US 5,330,428, Wang et al..

Examples of polyamides which may be used include nylon 6, nylon 64, nylon 66, nylon 610, nylon 610, nylon 612, nylon 46, nylon 9, nylon 10, nylon 11, nylon 12, and mixtures thereof.

The medical balloon article may be formed of polyurethanes such as Tecothane® from Thermedics. Tecothane® is a thermoplastic, aromatic, polyether polyurethane synthesized from methylene diisocyanate (MDI), polytetramethylene ether glycol (PTMEG) and 1,4 butanediol chain extender. Tecothane® 1065D and 1075D are examples. Other polyurethanes which have been used are Isoplast® 301, a high strength engineering thermoplastic polyurethane, and Pellethane® 2363-75D, both sold by Dow Chemical Co. References illustrating polyurethane balloon materials include US 4,950,239, to Gahara, US 5,500,180 to Anderson et al, US 6,146,356 to Wang, et al., and US 6,572,813, to Zhang, et al.

Balloons of the invention may be also made of polyamide/polyether block copolymers. The polyamide/polyether block copolymers are commonly identified by the acronym PEBA (polyether block amide). The polyamide and polyether segments of these block copolymers may be linked through amide linkages, however, most preferred are ester linked segmented polymers, i.e. polyamide/polyether polyesters. Such polyamide/polyether/polyester block copolymers are made by a molten state polycondensation reaction of a dicarboxylic polyamide and a polyether diol. The result is a short chain polyester made up of blocks of polyamide and polyether.

Polyamide/polyether polyesters are sold commercially under the Pebax® trademark by Elf Atochem North America, Inc., Philadelphia Pa. Examples of suitable commercially available polymers are the Pebax® 33 series polymers with hardness 60 and above, Shore D scale, especially Pebax® 6333, 7033 and 7233. These polymers are made up of nylon 12 segments and poly(tetramethylene ether) segments.

It is also possible to utilize polyester/polyether segmented block copolymers and obtain similar balloon properties. Such polymers are made up of at least two polyester and at least two polyether segments. The polyether segments are the same as previously described for the polyamide/polyether block copolymers useful in the invention. The polyester segments are polyesters of an aromatic dicarboxylic acid and a two to four carbon diol. The polyether segments of the polyester/polyether segmented block copolymers are aliphatic polyethers having at least 2 and no more than 10 linear saturated aliphatic carbon atoms between ether linkages. More preferably the ether segments have 4-6 carbons between ether linkages, and most preferably they are poly(tetramethylene ether) segments. Examples of other polyethers which may be employed in place of the preferred tetramethylene ether segments include polyethylene glycol, polypropylene glycol, poly(pentamethylene ether) and poly(hexamethylene ether). The hydrocarbon portions of the polyether may be optionally branched. An example is the polyether of 2-ethylhexane diol. Generally such branches will contain no more than two carbon atoms. The molecular weight of the polyether segments is suitably between about 400 and 2,500, preferably between 650 and 1000.

The polyester segments may be polyesters of an aromatic dicarboxylic acid and a two to four carbon diol. Suitable dicarboxylic acids used to prepare the polyester segments of the polyester/polyether block copolymers are ortho-, meta- or para-phthalic acid, napthalenedicarboxylic acid or meta-terphenyl-4,4'-dicarboxylic acids. Preferred polyester/polyether block copolymers are poly(butylene terephthalate)-block-poly(tetramethylene oxide) polymers such as Arnitel® EM 740, sold by DSM Engineering Plastics, and Hytrel® polymers, sold by DuPont, such as Hytrel 8230.

Examples of thermoplastic polyimides are described in T. L. St. Clair and H. D. Burks, "Thermoplastic/Melt-Processable Polyimides," NASA Conf. Pub. #2334 (1984), pp. 337-355. A suitable thermoplastic polyimide is described in U.S. Pat. No. 5,096,848 and is available commercially under the tradename Aurum® from Mitsui Toatsu Chemicals, Inc., of Tokyo, Japan.

Examples of liquid crystal polymers include the products Vectra® from Hoechst Celanese; Rodrun® from Unitika; LX and HX series polymers and Zenite™ polymers from DuPont; Sumikosuper™ and Ekonol™ from Sumitomo Chemical; Granlar™ from Grandmont; and Xydar® from Amoco. Suitably the liquid crystal polymer materials are blended with another thermoplastic polymer such as PET, nylon 12, or a block copolymer such as Pebax® 7033 or 7233 or Arintel® EM 740 or Hytrel 8230. In some cases the liquid crystal polymer may be present in a blend as fibers dispersed in a matrix.

Physical blends and copolymers of such materials may also be used.

Crystallization enhancers enhance crystallization, for instance by providing more effective nucleation sites, increasing crystallization rate or by other mechanisms, and crystallization inhibition inhibit crystallization, for instance by tying up nucleating sites or terminating crystal propagation or by some other mechanism.

As a general rule, to which exceptions occur, organic nucleating agents should have these attributes:
Crystal structure should be similar to that of the polymer. Increasing compatibility of crystal structure promotes crystal growth;
Nucleating agent should be insoluble in the polymer;
Melting point of nucleating agent should be above the melting point of the polymer;
Nucleating agent should be non-volatile and inert towards environment (polymer, oxygen, humidity, other additives, etc.); and
Nucleating agent should be well-dispersed in the polymer.

Table 2 provides examples of various types of polymers and crystallization enhancers which may be utilized therewith:

**Table 2**

| Polymer | Enhancers |
|---|---|
| Polyolefins | Organic or mineral nucleating agents, such as 1,2,3,4-bis-(3,4-dimethylbenzylidene sorbitol), methyldibenzylidene sorbitol, calcium stearate, Irgaclear® D, Irgaclear® DM , Irgaclear® B 215, Milad® 3988 |
| Polyesters and polyester block copolymers | Diphenylketone |
| | Eu(acac)₃•diPy |
| | Mn(CH₃COO)₂ + SbO₃ |
| | Selar® resins (PET polyolefin blends) |
| Polyamides; | Polyamide/(acrylonitrile-butadiene-styrene terpolymer) |
| Poly(amide-ether) block copolymers; | Polyamide/(styrene-acrylonitrile copolymer) |
| Poly(amide-ether-ester) block copolymers | |

Table 3 provides citations to examples of nucleating agents for polymer materials which occur in the open literature.

**Table 3**

| nucleating agent | polymer | reference | year | vol | no | page(s) |
|---|---|---|---|---|---|---|
| 1,2,3,4-bis-(3,4-dimethylbenzylidene sorbilol) | polypropylene (PP) | Journal of Applied Polymer Science | 2002 | 84 | | 2440-2450 |
| 1,3:2,4-Bis-(m-methylbenzylidene) sorbitol | polypropylene (PP) | Macromolecular Symposium | 2001 | 176 | | 63-91 |
| calcium stearate | polypropylene (PP) | Macromolecular Symposium | 2001 | 176 | | 83-91 |
| CrO3/SiO2/SI | polyethylene | Macromolecules | 1999 | 32 | | 8910-8913 |
| diphenylketone | crystalline copolymers based on poly(ethylerte terephthalate) | Journal of Applied Polymer Science | 2001 | 79 | | 497-503 |
| Eu(acac)3.diPy | poly(ethylene terephthalate | Polymer | 1997 | 38 | 17 | 4469-4476 |
| Irgaclear® D | polypropylene (PP) | Macromolecular Symposium | 2001 | 176 | | 83-91 |
| Irgaclear® OM | polypropylene (PP) | Macromolecular Symposium | 2001 | 176 | | 83-91 |
| Irganox® B 215 (Irgafos® 168:Irganox® 1010 = 2:1) | polypropylene (PP) | Macromolecular Symposium | 2001 | 176 | | 83-91 |
| liquid crystalline polymer | maleic anhydride-grafted polypropylene (m-PP) | Journal of Applied Polymer Science | 1996 | 64 | | 707-715 |
| methyldibenzylidene sorbitol | polypropylene (PP) | Journal of Applied Polymer Science | 2002 | 84 | | 2440-2450 |
| Millad 3988 | polypropylene (PP) | Macromolecular Symposium | 2001 | 176 | | 83-91 |
| Mn(CH3COO)2 + Sb203 | poly(ethylene terephthalate | Polymer | 1997 | 38 | 17 | 4469-4476 |
| PA6/acrylonitrile-butadiene- | polyamide 6 (PA6) | Journal of Applied Polymer Science | 2002 | 84 | | 2753-2759 |
| styrene terpolymer (ABS) | | | | | | |
| PA6/styrene-acrylonitrile copolymer (SAN) | polyamide 6 (PA6) | Journal of Applied Polymer Science | 2002 | 84 | | 2753-2759 |
| poly(L-lactide) (PLLA) | poly(L-lactide) poly(D-lactide) stereocomplex | Journal of Polymer Science: Part B: Physics Polymer | 2001 | 39 | | 300-313 |
| residual metatiocence catalysts | syndiotactic Polypropylene (sPP) | Journal of Applied Polymer Science | 2000 | 75 | | 337-346 |
| Sm(CH3COO)3. xH2O | poly(ethylene terephthalate | Polymer | 1997 | 38 | 17 | 4469-4476 |
| sodium acetate | crystalline copolymers based on poly(ethylene terephthalate) | Journal of Applied Polymer Science | 2001 | 79 | | 497-503 |
| sodium benzoate | crystalline copolymers based on poly(ethylene terephthalate) | Journal of Applied Polymer Science | 2001 | 79 | | 497-503 |
| sodium benzoate | nylon | Makromoleciile Band 1+2 Technologie, 5th Edition | 1994 | | | 34 |
| sodium benzoate | polypropylene (PP) | Makromolec0le Band 1+2 Technologie, 1994 5th Edition | | | | 34 |
| sodium stearate | glass-filled poly(propylene terephthalate) (GF PPT) | Journal of Applied Polymer Science | 1999 | 74 | | 889-899 |
| styrene-acrylonitrile-maleic anhydride copolymer (SAN MA) | polyamide 6 (PA6) | Journal of Applied Polymer Science | 2002 | 84 | | 2753-2759 |
| substituted sorbitol acetals | polypropylene (PP) | Journal Polymer Science, Polymer Letters | 1983 | 21 | | 34 |
| substituted sorbitol acetals | polypropylene (PP) | Macromolecular Symposium | 2001 | 176 | | 83-91 |
| substituted sorbitol acetals | polypropylene (PP) | Progress in Colloidial Polymer Science | 1992 | 87 | | 2 |
| talc | glass-filled poly(propylene terephthalate) (GF PPT) | Journal of Applied Polymer Science | 1999 | 74 | | 889-899 |
| talc | poly(L-lactide) poly(D-lactide) stereocomplex | Journal of Polymer Science: Part B: Polymer Physics | 2001 | 39 | | 300-313 |
| talc | polypropylene (PP) | Journal of Applied Polymer Science | 2002 | 84 | | 2440-2450 |
| talc & other mineral fillers | nylon | Kunsistoffe Aktuell | 1973 | 27 | | 10 |
| talc & other mineral fillers | PBT | Kunststoffe Aktuell | 1973 | 27 | | 10 |
| Tb(acac)3.diPy | poly(ethylene terephthalate | Polymer | 1997 | 38 | 17 | 4469-4476 |
| Ti(Q-n-C4H9)4 | poly(ethylene terephthalate | Polymer | 1997 | 38 | 17 | 4469-4476 |

A polymer system employing a crystallization inhibitor is described in US 5306246 (Sahatjian) in which PET/polyolefin blends (solar® resins) are added to PET in amounts up to 20% by weight of the composition.

Another polymer system employing a crystallization inhibitor is described in WO 94/21726 in which single-layer oriented heat-shrinkable films are obtained from polymer compositions comprising an ethylene/α-olefin copolymer, a polymeric alloy (made up of a heterophasic composition in which an amorphous ethylene/propylene copolymer is dispersed in a homopolymeric propylene matrix) and/or a random copolymer of propylene with ethylene, and a crystallization inhibitor For this purpose the crystallization inhibitor may be one or more of aliphatic and aromatic hydrocarbon resins, aliphatic and aromatic copolymers, such as polymers and copolymers of piperylene, methylbutene, isobutene, vinyltoluene, indene, α-methylstyrene, polycyclodiene, etc.; hydrogenated C₉ resins; and pinene and rosin resins and terpene resins.

Nucleating agents which may be employed in the compositions as crystallization enhancers are known, for instance from US 20030054161, US 20030148056, and US 6610765. As reported in these documents nucleating agents which have been used previously for polymer films include mineral nucleating agents and organic nucleating agents. Examples of mineral nucleating agents include carbon black, silica, kaolin, sodium bicarbonate and talc. Among the organic nucleating agents which have been suggested as useful in polyolefin films include salts of aliphatic monobasic or di-basic acids or arylalkyl acids such as sodium succinate, sodium glutarate, sodium caproate, sodium 4-methylvalerate, sodium-2-2'-methylenebis(4,6-di-tert-butylphenyl)phosphate, aluminum phenyl acetate, and sodium cinnamate. Alkali metal and aluminum salts of aromatic and alicyclic carboxylic acids such as aluminum benzoate, sodium or potassium benzoate, sodium beta-naphtholate, lithium benzoate and aluminum tertiary-butyl benzoate also are useful organic nucleating agents. The free acids of the above mentioned salts may also be suitable. Benzenesulfonamides have been reported to be useful nucleating agents, as well as substituted sorbitol derivatives such as bis-(benzylidene) and bis-(alkylbenzilidine) sorbitols wherein the alkyl groups contain from about four to about eighteen carbon atoms. Particular such substituted sorbitol derivatives are compounds of formula I 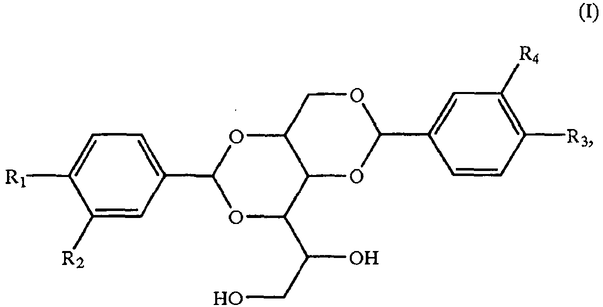 wherein R₁, R₂, R₃ and R₄ are each independently of one another hydrogen or C₁ - C₄ alkyl, and C₁ - C₄ alkyl is a branched or unbranched radical, for example methyl, ethyl, n-propyl, isopropyl, n-butyl, isobutyl or tert-butyl. Specific nucleating agents are the compounds of formula la (Irgaclear® DM), 1b (Irgaclear® D) and 1c (Millad® 3988). 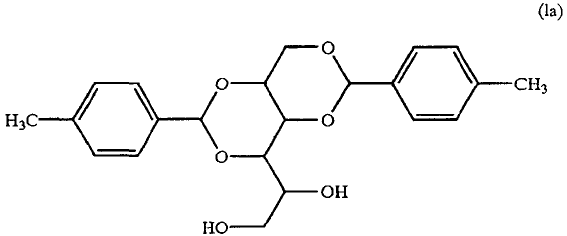 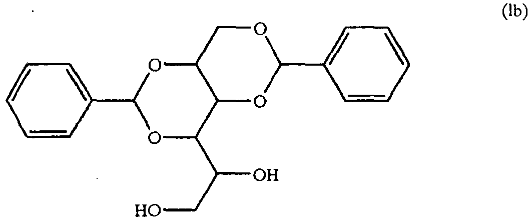 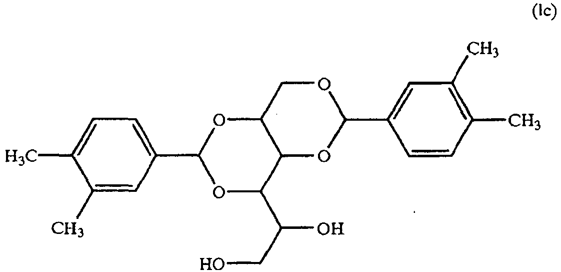 Irgaclear® DM and Irgaclear® D are registered trademarks of Ciba Spezialitatenchemie AG. Millad® 3988 is a registered trademark of Milliken & Company. Mixtures of any of the nucleating agents may be used, for instance a mixture of Irgaclear® DM and sodium benzoate. Other nucleating agents which can be used include phosphate ester based products such as NA-11 and NA-21 supplied by Asahi-Denka Kogyo of Japan, and a norbomane carboxylic acid salt based product HPN-68 supplied by Milliken & Company.

The nucleating agents described above are typically used with polyolefins, especially polypropylene polymers and copolymers, but in some polyethylene polymers or copolymers. Similar nucleating agents can be used for other semi-crystalline polymers.

The amounts of crystallization modifier incorporated into the formulations of the present invention will vary depending on their impact on the specific polymer employed in the composition and the degree of crystallization modification desired. In some cases quite small amounts, for instance as low as 100 ppm, may be suitable, especially in the case where the modifier is a nucleating agent, while in other cases amounts in excess of 3%, for instance up to 20% by weight may be suitable, particularity in the case of polymer or resinous crystallization inhibitors or up to 10 % by weight in the case of nucleating agents. In some cases the maximum desirable amount will be in the range of from 0.1-1% by weight.

In accordance with the invention, varying the amount of the crystallization modifier by location within the inventive device part allows a more effective tailoring of properties of such part to localized differences in desired properties such as strength, softness, flexibility, distension and the like. The variation may be step-wise, or continuous, and it may range from zero to some positive amount, or between positive amounts. For example the modifier may be varied from zero to about 20% by weight of the polymer composition, from zero to about 10%, from 0.5% to about 5%, from 100 ppm to 2000 ppm, or from zero to about 3% by weight of the polymer composition.

A single medical device part may also be described with more than one crystallization rate modifier. For instance a catheter inner shaft may be formed from a polymer composition comprising a crystallization rate enhancer in a proximal region, the enhancer tapering to zero moving distally. Then, at the distal end, a crystallization rate inhibitor may be incorporated into the composition. A polymer composition incorporating a crystallization enhancer may be used to form a catheter outer in the proximal region, the composition having a lesser or no amount of enhancer in an intermediate region and then once again incorporate an enhancer just proximal of the distal end to increase yield strength and thereby enhance resistance to necking during withdrawal of the catheter.

The composition variation employed in the invention can be coupled with concurrent complementary variations in extrusion or injection parameters which alter the available crystallization time, or device profile, to further increase the difference proximal to distal in stiffness, flexibility and/or other crystallization related physical properties. For instance a crystallization enhancer may be incorporated into an extrusion melt at the same time that the tank gap is changed to enhance longitudinal orientation and/or the tube diameter or wall thickness is increased. Crystalline structure in the formed part stabilizes polymer orientation obtained from processing operations such as extrusion, stretching, and parison blow-forming techniques, reducing creep relaxation which may occur over time or as a result of use stress.

Where the desired locality of the modified composition is small, extrusion and injection systems which allow change-over from one composition to another using very low volumes are preferably used. Where a gradual transition in properties is desired a wider range of composition supply systems can be used. Co-injection molding, gradient extrusion, coextrusion, and intermittent extrusion equipment are examples of supply systems which may be used.

The invention has application to the preparation of preformed balloon parisons, for providing a crystallization inhibitor in the distal and/or proximal waist region to reduce crystallization during laser welding of the balloon to the catheter. Concurrently, or alternatively, in the portion of the parison used to form balloon body region a crystallization enhancer may be employed to reduce creep behavior and enhance the elastic response of the balloon after a first inflation.

In a multi-layer laminate catheter or balloon, crystallization modifiers may be employed in one or multiple layers. This may be desirable, for instance, to increase or decrease selected property differences between the two layers.

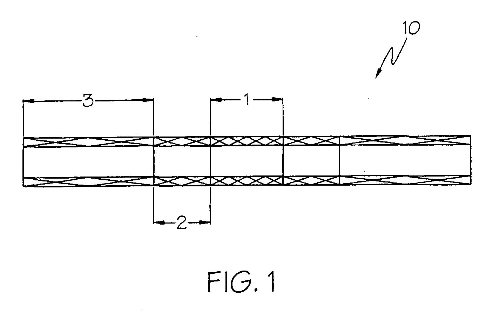

Referring to the figures, Figure 1 shows an extruded balloon parison 10 prepared in accordance with an aspect of the invention, with crosshatching indicating the variation in composition. The segment has three distinct regions 1, 2, 3, each with different levels of crystallization. Region 3 has a crystallization inhibitor incorporated into the polymer composition and will produce a balloon waist portion which undergoes very little crystallization as a consequence of heat bonding to the catheter distal tip. Region 2 is a transition region as the composition changes over to an unmodified polymer composition in region 1. In subsequent processing operations region 1 will form the balloon body, region 2, will form the proximal cone, or a distal portion thereof, and region 3 will form a waist portion of the balloon.

A balloon having some crystallization inhibitor in the cone region may be desirable, for instance, to improve re-inflation cycle integrity of balloons which are heat set. Heat setting, at a temperature above the blowing temperature, increases polymer crystallization after the balloon is initially formed. Heat setting increases balloon inflation strength, but can reduce ability of the balloon to under go repeated inflations to dilatation pressures without failure. Failure in the cone regions has been observed. Using a crystallization inhibitor in the cone region but not in the balloon body portion, when forming a heat set balloon, can allow the increased burst strength advantages of heat set balloons to be retained while the disadvantage of reduced re-inflation cycle integrity is minimized or eliminated.

More complex patterns are also available. Stepped transitions may be produced, stepping progressively up or down, or both up and down in crystallization modifier, the length of transition region(s) may be different for different steps, or the variation in composition may be continuous. For instance using a single polymer, continuous variation of modifier component of the polymer composition over an elongated region of a catheter shaft may displace the need to manufacture the shaft in two or more segments of different polymers.

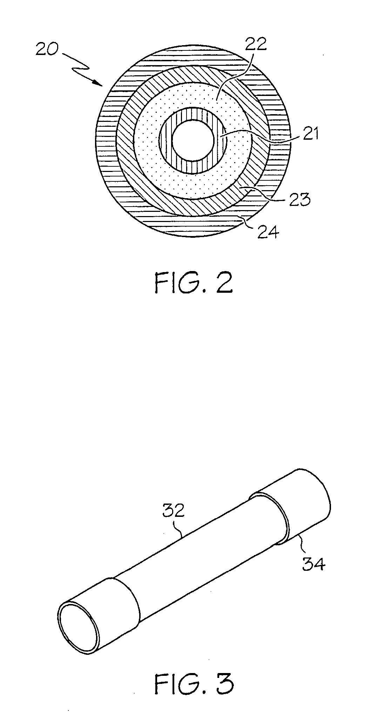

In a multi-layer laminate catheter or balloon a crystallization modifier may be employed to increase property differences in the two layers. Figure 2 shows a cross-sectional view of a multi-layer tube 20 which is formed of layers 21, 22, 23, and 24 which may all be formed of the same polymer, but differing polymer compositions due to the presence and/or amount of crystallization inhibitor or enhancer. The crystallinity variation may step up or down linearly as one passes through the thickness dimension of the tube, it may alternate between layers of lower crystallization and higher crystallization, or it may follow some other pattern.

Figure 3 depicts a tube 32, which may be, for instance a catheter tube segment 32, or a balloon parison. Tube 32 includes layer 34 deposited by an intermittent extrusion, in selected regions. Layer 34 may be substantially the same polymer material as the underlying tube, except that it is provided with a crystallization modifier. For instance in the case of a catheter tube, the layer may be a crystallization inhibitor, deposited at fusion bonding sites, such as where the balloon is bonded to the shaft, so as to reduce crystallization, and resulting stiffening at those sites as a result of the fusion bonding step.

Differences in polymer modulus of as much as four times may be produced, relative to unmodified polymer, simply by the incorporation of an optimal amount of a crystallization inhibitor or enhancer. Even larger differences may be obtainable if the composition transitions from incorporation of an enhancer in one portion of the device to an inhibitor in another portion.

In a catheter shaft application, selective crystallization and tube wall reduction could enable a continuous tapered shaft from one material without the need for distal shaft bonds. This can be coupled with concurrent complementary variations in extrusion parameters which alter the available crystallization time, to further increase the difference proximal to distal in stiffness, flexibility and/or other crystallization related physical properties.

## Claims

1. A medical balloon formed of a tubular balloon parison, the tubular balloon parison formed of a melt processed polymer material composition, the polymer material composition comprising at least one crystallizable base polymer and, in at least a portion of the tubular parison further comprising a crystallization modifier, wherein from a first portion (1, 32) of the tubular parison to a second portion (2, 32) of the tubular parison, the polymer material composition, is varied in the amount of the crystallization modifier relative to the amount of said at least one crystallizable base polymer, in the portion of the tubular parison used to form the distal and/or proximal end is provided a crystallization inhibitor.

2. A medical balloon as in claim 1 wherein said crystallizable base polymer is selected from the group consisting of olefin, acrylic, styrenic and vinyl polymers and copolymers; polyethers; polyamides; polycarbonates: polyesters; polyurethanes; thermoplastic polyimides; liquid crystal polymers; ABS (acrylonitrile butadiene styrene); ANS (acrylonitrile styrene); polyacetal; PEI (polyetherimide); polyetheretherketone (PEEK); and polyether sulfone (PES); block copolymers comprising at least one polyolefin, polyacrylic, polystyrenic, polyvinyl, polyether, polyamide, polyester, or polyurethane block therein, and mixtures of any of said polymers.

3. A medical balloon as in claim 1 wherein the medical balloon is a dilatation balloon.

4. A medical balloon as in claim 3 comprising a balloon body portion and proximal and distal waist portions, wherein the crystallization modifier is present in the distal waist portion of the balloon.

5. A medical balloon as in claim 4 wherein the crystallization modifier is not present in the balloon body portion of the balloon.

6. A medical balloon as in claim 1 wherein said balloon is a discreetly formed portion of a balloon catheter.

7. A method of forming the medical balloon according to claim 1 comprising passing a mass of molten polymer material composition through an opening to form an emitted mass, the polymer material composition comprising at least one crystallizable base polymer and, in at least a portion of the part further comprising a crystallization modifier,
subsequently cooling the emitted mass, without mixing the emitted mass material, whereby the cooled emitted mass comprises at least two regions of material located within the cooled mass in a fixed relationship to each other said fixed relationship corresponding to the sequence of emission of the polymer material forming each said region,
wherein the method further comprises
varying an amount of crystallization modifier in the polymer composition passing through said opening between the emission of the material forming the first region and the emission of the material forming the second region, whereby at least one of the two regions is provided with a positive amount of said crystallization modifier and the two regions are provided with differing amounts of said crystallization modifier,
wherein the crystallization modifier is a crystallization inhibitor.

8. The method of claim 7 wherein the passing step comprises extruding said molten polymer composition through a die head.

9. The method of claim 8 wherein the passing step comprises injecting the polymer mass into a mold form.

10. The method of claim 9 wherein said polymer composition comprises a crystallizable base polymer selected from the group consisting of olefin, acrylic, styrenic and vinyl polymers and copolymers; polyethers; polyamides; polycarbonates; polyesters; polyurethanes; thermoplastic polyimides; liquid crystal polymers, ABS (acrylonitrile butadiene styrene); ANS (acrylonitrile styrene); polyacetal; PEI (polyetherimide); polyetheretherketone (PEEK); and polyether sulfone (PES); block copolymers comprising at least one polyolefin, polyacrylic, polystyrenic, polyvinyl, polyether, polyamide, polyester, or polyurethane block therein, and mixtures of any of said polymers.

## Patentansprüche

1. Medizinischer Ballon, der aus einem schlauchförmigen Ballonvorförmling geformt ist, wobei der schlauchförmige Ballonvorförmling aus einer schmelzverarbeiteten Polymermaterialzusammensetzung geformt ist, wobei die Polymermaterialzusammensetzung mindestens ein kristallisierbares Basispolymer umfasst und in mindestens einem Teil des schlauchförmigen Vorförmlings des Weiteren einen Kristallisationsmodifikators umfasst, wobei von einem ersten Teil (1, 32) des schlauchförmigen Vorförmlings zu einem zweiten Teil (2, 32) des schlauchförmigen Vorförmlings die Polymermaterialzusammensetzung im Hinblick auf die Menge des Kristallisationsmodifikators in Bezug auf die Menge des mindestens einen kristallisierbaren Basispolymers variiert ist, wobei in dem Teil des schlauchförmigen Vorförmlings, der zum Formen des distalen und/oder proximalen Endes verwendet wird, ein Kristallisationshemmer bereitgestellt ist.

2. Medizinischer Ballon nach Anspruch 1, wobei das kristallisierbare Basispolymer ausgewählt ist aus der Gruppe, bestehend aus Olefin, Acryl-, Styrol- und Vinylpolymeren und -copolymeren; Polyethern; Polyamiden; Polycarbonaten; Polyestern; Polyurethanen; thermoplastischen Polyimiden; Flüssigkristallpolymeren; ABS (Acrylnitril-Butadien-Styrol); ANS (Acrylnitril-Styrol); Polyacetal; PEI (Polyetherimid); Polyetheretherketon (PEEK); und Polyethersulfon (PES); Blockcopolymeren, umfassend mindestens einen Polyolefin-, Polyacryl-, Polystyrol-, Polyvinyl-, Polyether-, Polyamid-, Polyester- oder Polyurethanblock darin, und Gemischen von beliebigen der Polymere.

3. Medizinischer Ballon nach Anspruch 1, wobei es sich bei dem medizinischen Ballon um einen Dehnballon handelt.

4. Medizinischer Ballon nach Anspruch 3, umfassend einen Ballonkörperteil und ein proximales und ein distales Mittelteil, wobei der Kristallisationsmodifikator im distalen Mittelteil des Ballons vorliegt.

5. Medizinischer Ballon nach Anspruch 4, wobei der Kristallisationsmodifikator nicht im Ballonkörperteil des Ballons vorliegt.

6. Medizinischer Ballon nach Anspruch 1, wobei es sich bei dem Ballon um einen gesondert geformten Teil eines Ballonkatheters handelt.

7. Verfahren zum Formen des medizinischen Ballons nach Anspruch 1, umfassend das Leiten einer Masse aus geschmolzener Polymermaterialzusammensetzung durch eine Öffnung zum Bilden einer Austrittmasse, wobei die Polymermaterialzusammensetzung mindestens ein kristallisierbares Basispolymer umfasst und in mindestens einem Teil des Teils des Weiteren einen Kristallisationsmodifikator umfasst,
anschließend Abkühlen der Austrittmasse ohne Mischen des Austrittmassenmaterials, wodurch die abgekühlte Austrittmasse mindestens zwei in der abgekühlten Austrittmasse in einem festgelegten Verhältnis zueinander lokalisierte Materialbereiche umfasst, wobei das festgelegte Verhältnis der Austrittsequenz des die jeweilige Region bildenden Polymermaterials entspricht,
wobei das Verfahren des Weiteren umfasst:
Variieren einer Menge an Kristallisationsmodifikator in der durch die Öffnung laufenden Polymerzusammensetzung zwischen dem Austritt des den ersten Bereich bildenden Materials und dem Austritt des den zweiten Bereich bildenden Materials, wodurch mindestens einer der beiden Bereiche mit einer positiven Menge des Kristallisationsmodifikators versehen wird und die beiden Bereiche mit unterschiedlichen Mengen des Kristallisationsmodifikators versehen werden,
wobei es sich bei dem Kristallisationsmodifikator um einen Kristallisationshemmer handelt.

8. Verfahren nach Anspruch 7, wobei der Durchleitschritt das Extrudieren der geschmolzenen Polymerzusammensetzung durch einen Düsenkopf umfasst.

9. Verfahren nach Anspruch 8, wobei der Durchleitschritt das Einspritzen der Polymermasse in eine Gussform umfasst.

10. Verfahren nach Anspruch 9, wobei die Polymerzusammensetzung ein kristallisierbares Basispolymer umfasst, das ausgewählt ist aus der Gruppe, bestehend aus Olefin, Acryl-, Styrol- und Vinylpolymeren und -copolymeren; Polyethern; Polyamiden; Polycarbonaten; Polyestern; Polyurethanen; thermoplastischen Polyimiden; Flüssigkristallpolymeren; ABS (Acrylnitril-Butadien-Styrol); ANS (Acrylnitril-Styrol); Polyacetal; PEI (Polyetherimid); Polyetheretherketon (PEEK); und Polyethersulfon (PES); Blockcopolymeren, umfassend mindestens einen Polyolefin-, Polyacryl-, Polystyrol-, Polyvinyl-, Polyether-, Polyamid-, Polyester- oder Polyurethanblock darin, und Gemischen von beliebigen der Polymere.

## Revendications

1. Ballonnet à usage médical formé d'une paraison tubulaire de ballonnet, la paraison tubulaire de ballonnet étant formée d'une composition d'un matériau polymère transformé à l'état fondu, la composition de matériau polymère comprenant au moins un polymère de base cristallisable et, dans au moins une portion de la paraison tubulaire, comprenant en outre un agent modifiant la cristallisation, dans lequel, d'une première portion (1, 32) de la paraison tubulaire à une deuxième portion (2, 32) de la paraison tubulaire, on fait varier dans la composition de matériau polymère la quantité de l'agent modifiant la cristallisation par rapport à la quantité dudit ou desdits polymères de base cristallisables, un inhibiteur de cristallisation étant prévu dans la portion de la paraison tubulaire utilisée pour former l'extrémité distale et/ou l'extrémité proximale.

2. Ballonnet à usage médical selon la revendication 1, dans lequel le polymère de base cristallisable est choisi dans le groupe consistant en les polymères et copolymères oléfiniques, acryliques, styréniques et vinyliques ; les polyéthers ; les polyamides ; les polycarbonates ; les polyesters ; les polyuréthannes ; les polyimides thermoplastiques ; les polymères cristaux liquides ; l'ABS (acrylonitrile-butadiène-styrène) ; l'ANS (acrylonitrile-styrène) ; le polyacétal ; le PEI (polyétherimide) ; la polyétheréthercétone (PEEK) ; et la polyéthersulfone (PES) ; les copolymères à blocs comprenant au moins un bloc polyoléfinique, polyacrylique, polystyrénique, polyvinylique, polyéther, polyamide, polyester ou polyuréthanne, et les mélanges de deux ou plusieurs quelconques de ces polymères.

3. Ballonnet à usage médical selon la revendication 1, le ballonnet à usage médical étant un ballonnet de dilatation.

4. Ballonnet à usage médical selon la revendication 3, comprenant une portion formant corps de ballonnet et des portions formant étranglement proximal et distal, l'agent modifiant la cristallisation étant présent dans la portion formant étranglement distal du ballonnet.

5. Ballonnet à usage médical selon la revendication 4, dans lequel l'agent modifiant la cristallisation n'est pas présent dans la portion formant corps de ballonnet du ballonnet.

6. Ballonnet à usage médical selon la revendication 1, ledit ballonnet étant une portion discrètement formée d'un cathéter à ballonnet.

7. Procédé de formation d'un ballonnet à usage médical selon la revendication 1, comprenant le passage d'une masse d'une composition de matériau polymère fondu à travers une ouverture pour former une masse émise, la composition de matériau polymère comprenant au moins un polymère de base cristallisable et, dans au moins une portion de la pièce, comprenant en outre un agent modifiant la cristallisation ; puis le refroidissement de la masse émise, sans mélange du matériau de la masse émise, la masse émise refroidie comprenant au moins deux régions de matériau situées à l'intérieur de la masse refroidie selon une relation réciproque fixe, ladite relation fixe correspondant à la séquence d'émission du matériau polymère formant chacune desdites régions ; ledit procédé consistant en outre à faire varier une quantité de l'agent modifiant la cristallisation dans la composition polymère passant par ladite ouverture entre l'émission du matériau formant la première région et l'émission du matériau formant la deuxième région, au moins l'une des deux régions étant ainsi pourvue d'une quantité positive dudit agent modifiant la cristallisation, et les deux régions étant pourvues de quantités différentes dudit agent modifiant la cristallisation ; ledit agent modifiant la cristallisation étant un inhibiteur de cristallisation.

8. Procédé selon la revendication 7, dans lequel l'étape de passage comprend l'extrusion de ladite composition de polymère fondu à travers une tête de filière.

9. Procédé selon la revendication 8, dans lequel l'étape de passage comprend l'injection de la masse polymère dans une empreinte de moule.

10. Procédé selon la revendication 9, dans lequel ladite composition polymère comprend un polymère de base cristallisable choisi dans le groupe consistant en les polymères et copolymères oléfiniques, acryliques, styréniques et vinyliques ; les polyéthers ; les polyamides ; les polycarbonates ; les polyesters ; les polyuréthannes ; les polyimides thermoplastiques ; les polymères cristaux liquides ; l'ABS (acrylonitrile-butadiène-styrène) ; l'ANS (acrylonitrile-styrène) ; le polyacétal ; le PEI (polyétherimide) ; la polyétheréthercétone (PEEK) ; et la polyéthersulfone (PES) ; les copolymères à blocs comprenant au moins un bloc polyoléfinique, polyacrylique, polystyrénique, polyvinylique, polyéther, polyamide, polyester ou polyuréthanne, et les mélanges de deux ou plusieurs quelconques de ces polymères.
